# DEMANDE DE BREVET EUROPEEN

(11) **EP 4 659 800 A1**
(43) Date de publication de la demande: **10.12.2025**
(21) Numéro de dépôt: 25179332.9
(22) Date de dépôt: 28.05.2025
(51) Int. Cl.: A61N 5/06

(54) **DISPOSITIF POUR EXPOSER UN CORPS HUMAIN OU ANIMAL À UN RAYONNEMENT LUMINEUX OU INFRAROUGE**

(30) Priorité: 07.06.2024 FR 2406001
(71) Demandeur: Xbiotec, 38110 Saint-Clair-de-la-Tour (FR)
(72) Inventeur: HOUOT, Jean-Laurent, 38110 Saint-Clair-de-la-Tour (FR); LEROY, Sébastien, 38110 La Chapelle-de-la-Tour (FR)
(74) Mandataire: IXAS Conseil

(57) **Abrégé**

Dispositif (10) pour exposer un corps humain (910) ou animal à un rayonnement lumineux ou infrarouge, qui comporte une pluralité de sources de rayonnement, chacune comportant au moins une diode électroluminescente disposée pour exposer à un rayonnement une partie du corps (911, 912, 913, 914) positionnée face à la source de rayonnement, dans lequel chaque source de rayonnement, comporte un capteur de distance configuré pour estimer une valeur représentative de la distance séparant la source de rayonnement et la partie du corps exposée à ladite source de rayonnement, et dans lequel l'intensité de l'irradiation lumineuse ou infrarouge émise par chaque source de rayonnement est déterminée en fonction de la distance séparant ladite source de rayonnement et la partie du corps exposée à ladite source de rayonnement. Procédé d'utilisation d'un tel dispositif.

## Description

### DOMAINE TECHNIQUE DE L'INVENTION

La présente demande concerne un dispositif pour exposer un corps humain ou animal à un rayonnement lumineux ou infrarouge, et plus particulièrement un dispositif présentant une pluralité de sources de rayonnement et dans lequel l'intensité de l'irradiation émise par chaque source de rayonnement est déterminée en fonction de la distance la séparant de la partie du corps exposée.

La présente demande concerne également un procédé de fonctionnement d'un tel dispositif.

### ÉTAT DE LA TECHNIQUE

L'art antérieur connaît des appareils comportant une multitude de diodes électroluminescentes, abrévié DEL, ou plus communément LED (de l'anglais Light-emitting diode) utilisées notamment dans le cadre de traitement de l'humain ou de l'animal.

La photobiomodulation fait partie des méthodes de traitement nécessitant un tel appareil. Il s'agit d'une technique nécessitant l'émission d'ondes électromagnétiques sur le corps et visant, entre autres, à favoriser la régénération cellulaire. Cette émission d'ondes parfois appelée « lumière froide » implique l'émission d'ondes de diverses longueurs, selon les types de traitement, qui pénètrent plus ou moins profondément dans la peau dans le but d'y susciter une réaction biochimique, par exemple en influant le fonctionnement des mitochondries et la chaîne respiratoire qui s'y déroule. La photobiomodulation implique généralement l'application d'une lumière monochromatique, ou d'un rayonnement infrarouge, produite par des LED sur une zone ou plusieurs zones du corps prédéfinies.

La société demanderesse a constaté que les appareils disponibles sur le marché ne permettaient pas de délivrer une dose d'exposition optimale, car ces appareils ne tiennent pas compte de la morphologie de l'utilisateur. Ainsi, l'intensité de rayonnement, qui est le plus souvent délivrée uniformément par les LED, est trop forte sur certaines parties du corps et trop faible sur d'autre. Ce problème est en outre aggravé lorsque l'utilisateur ne se place pas dans une position optimale par rapport à l'appareil comportant les LED.

### OBJETS DE L'INVENTION

La présente invention vise à améliorer les dispositifs connus de l'art antérieur en modulant l'intensité du rayonnement émis sur l'utilisateur par chaque source de rayonnement en fonction de la distance de cette source par rapport à la partie du corps de l'utilisateur qu'elle irradie.

À cet effet, selon un premier aspect, l'invention vise un dispositif pour exposer un corps humain ou animal à un rayonnement lumineux (typiquement dans le spectre visible ou infrarouge), qui comporte une pluralité de sources de rayonnement, chacune comportant au moins une diode électroluminescente disposée pour exposer à un rayonnement une partie du corps humain ou animal positionnée face à la source de rayonnement,
dans lequel chaque source de rayonnement, comporte un capteur de distance configuré pour estimer une valeur représentative de la distance séparant la source de rayonnement et la partie du corps exposée à ladite source de rayonnement,
et dans lequel l'intensité de l'irradiation lumineuse ou infrarouge émise par chaque source de rayonnement est déterminée en fonction de la distance séparant ladite source de rayonnement et la partie du corps exposée à ladite source de rayonnement.

Préférentiellement, chaque source de rayonnement comporte une pluralité de diodes électroluminescentes (ci-après abrévié LED), par exemple arrangées en ligne ou en un groupement relativement compact. Avantageusement, la pluralité de LED formant une source de rayonnement sont arrangés pour que chacune des LED se trouve à une distance sensiblement identique d'une partie du corps exposé la source de rayonnement, lors d'une utilisation normale du dispositif.

Grâce aux dispositions de l'invention, l'intensité de rayonnement irradiant chaque zone du corps est modulée en fonction de la distance séparant cette zone du corps et la source de rayonnement. En effet, la LED ou l'ensemble de LED formant chaque source de rayonnement est dotée d'un capteur de distance et l'intensité lumineuse ou infrarouge délivrée par lesdites LED est ajustée en fonction de la distance mesurée.

Par ailleurs, l'intensité de rayonnement pourra être automatiquement ajustée en fonction d'une sélection faite par l'utilisateur telles que la sélection d'un traitement souhaité ou en fonction de caractéristiques morphologiques de l'utilisateur, telles que sa taille ou sa carnation.

Dans des modes de réalisation (non représentés), le dispositif comporte un capteur configuré pour estimer la carnation de l'utilisateur et l'intensité de rayonnement produite par les sources de rayonnement est déterminée en fonction de la carnation estimée.

Dans des modes de réalisation, le dispositif comporte une pluralité de rangées de diodes électroluminescentes disposées horizontalement les unes au-dessus des autres, chaque rangée étant associée à un capteur de distance positionné sensiblement à la même hauteur que la rangée de diodes électroluminescentes et chaque rangée formant une source de rayonnement.

Grâce à ces dispositions, le nombre de capteurs de distance peut être limité tout en conservant une bonne estimation de la corpulence et/ou de la posture de l'utilisateur du dispositif.

Dans des modes de réalisation, le dispositif comporte un moyen d'indication d'une position où doit être placé le corps humain ou animal lors de l'utilisation du dispositif et la position indiquée par le moyen d'indication d'une position est mobile, permettant d'indiquer plusieurs positions prédéterminées.

Par exemple, le moyen d'indication communique à l'utilisateur la place idéale ou positionner ses pieds en indiquant une marque au sol devant le dispositif. Selon un autre exemple, un message sonore est émis par un haut-parleur, indiquant à l'utilisateur de s'approcher ou de se reculer par rapport à l'appareil. Selon un autre exemple, un affichage informe l'utilisateur sur l'endroit où il doit se positionner. Toute autre moyen permettant de communiquer une information à l'utilisateur où il doit se positionner pourra être mis en œuvre. Dans des modes de réalisation, le moyen d'indication permet de communiquer cette information à un aidant ou personnel soignant qui aide l'utilisateur à se positionner. Dans des modes de réalisation, le moyen d'indication permet de communiquer cette information à un vétérinaire ou tout autre personne souhaitant positionner correctement le corps d'un animal par rapport au dispositif.

Grâce à ces dispositions, l'utilisateur peut connaître une position optimisée où se placer par rapport au dispositif. On entend par position optimisée une position qui permet une meilleure application de traitement par le dispositif, qui permet par exemple de positionner l'utilisateur dans l'intervalle optimal de fonctionnement des LED.

La position optimisée destinée à être indiquée par le moyen d'indication pourra être déterminée au moyen d'un microprocesseur, en tenant compte d'informations telles que les caractéristiques de l'utilisateur (âge, corpulence, carnation, etc.) du dispositif ou du type de traitement à réaliser. Ces informations pourront être saisies manuellement ou détectées au moyen de capteurs.

Dans des modes de réalisation, la position indiquée par le moyen d'indication d'une position est déterminée en fonction d'au moins une valeur représentative de la distance séparant le corps, ou une partie du corps, du dispositif.

Selon ce mode de réalisation, la position indiquée est dynamique, c'est-à-dire qu'elle est automatiquement modifiée en fonction de la distance estimée entre l'utilisateur et le dispositif. La position indiquée pourra être déterminée en début de session de traitement puis conservée tout au long de ce dernier, ou modifiée au cours du traitement, par exemple pour tenir compte d'un changement de la posture de l'utilisateur.

Grâce à ces dispositions, le gabarit, la corpulence ou encore la posture de l'utilisateur peuvent être pris en considération dans la détermination d'une position optimale à indiquer. Par exemple, un utilisateur de forte corpulence pourra se voir indiquer une position optimale légèrement en retrait par rapport au dispositif, de sorte que la distance entre les LED et l'utilisateur ne sera trop courte en aucun point. Au contraire, un utilisateur de faible corpulence pourra être invité à s'approcher des sources de rayonnement.

Dans des modes de réalisation, un capteur de distance dédié est consacré à l'estimation de la distance entre l'utilisateur et le dispositif. Dans des modes de réalisation préférentiels, un même capteur de distance ou un même ensemble de capteurs de distance est utilisé pour réaliser l'estimation de distance utilisée afin de moduler l'intensité lumineuse des moyens d'irradiation et pour réaliser l'estimation de distance utilisée pour déterminer la position où doit être placé le corps humain ou animal lors de l'utilisation du dispositif.

Dans des modes de réalisation, au moins un capteur de distance parmi ceux précités est du type choisi parmi : un capteur infrarouge estimant une distance en fonction de l'intensité de la lumière réfléchie, un capteur optique estimant une distance en fonction du temps de vol (capteur ToF, de l'anglais « Time Of Flight »), un capteur LIDAR (de l'anglais « Light Detection and Ranging »). Tout autre capteur connu de l'Homme du Métier et permettant de mesurer une distance entre le dispositif et l'utilisateur pourra être mis en œuvre.

Dans des modes de réalisation, le moyen d'indication d'une position comporte un moyen d'émission d'un faisceau laser configuré pour marquer une position, par exemple matérialisée par une ligne projetée au sol où un utilisateur du dispositif doit positionner ses pieds. Le faisceau laser est un moyen simple et compact permettant de tracer une ligne clairement visible à l'endroit où l'utilisateur est invité à positionner ses pieds.

Dans d'autres modes de réalisation, le moyen d'indication d'une position comporte un moyen d'affichage numérique. Par exemple, le moyen d'indication comporte un écran ou un vidéoprojecteur configuré pour afficher une image indiquant où un utilisateur du dispositif doit positionner ses pieds.

Grâce à ces dispositions, le vidéoprojecteur ou l'écran permet de projeter des images plus complexes, susceptibles d'apporter une information plus riche ou plus précise à l'utilisateur. Par exemple, l'image projetée ou affichée pourra indiquer à l'utilisateur la posture qu'il doit tenir, ou indiquer le temps de traitement restant, ou encore service d'affichage pour une interface homme-machine permettant d'interagir avec le dispositif.

Dans des modes de réalisation, le dispositif comporte un moyen de détection de la position de l'utilisateur.

Dans des modes de réalisation, le moyen de détection de la position de l'utilisateur détecte la position de l'utilisateur au moyen d'au moins un capteur de distance, préférentiellement au moyen de l'un au moins des capteurs de distance des sources de lumière.

Dans des modes de réalisation, le moyen de détection de la position de l'utilisateur comporte au moins un capteur de pression, préférentiellement une pluralité de capteurs de pression, disposés au sol devant les sources de rayonnement et configurés pour détecter la présence du corps humain ou animal.

Dans des modes de réalisation, le dispositif comporte un moyen de vérification configuré pour déterminer si la position d'un corps humain ou animal détectée par le moyen de détection correspond à la position où doit être placé le corps humain ou animal lors de l'utilisation du dispositif indiquée par le moyen d'indication d'une position.

Grâce à ces dispositions, si l'utilisateur n'est pas présent ou mal positionné par rapport au dispositif, une information peut lui être communiquée. Par exemple, une alarme sonore, visuelle ou silencieuse est émise dans le cas où le moyen de vérification ne parvient pas à confirmer la présence d'un corps à la position prévue par le moyen d'indication.

Dans des modes de réalisation, l'intensité de l'irradiation lumineuse ou infrarouge émise par au moins une source de rayonnement est déterminée en fonction de la vérification opérée par le moyen de vérification. Par exemple, dans le cas où le moyen de vérification ne parvient pas à confirmer la présence d'un corps à la position prévue par le moyen d'indication, l'intensité de l'irradiation lumineuse ou infrarouge émise par au moins une source de rayonnement est réduite à une très basse intensité ou est éteinte.

Selon un deuxième aspect, l'invention vise un procédé de fonctionnement d'un dispositif pour exposer un corps humain ou animal à un rayonnement lumineux ou infrarouge comportant une pluralité de sources de rayonnement, chacune comportant au moins une diode électroluminescente disposée pour exposer à un rayonnement une partie du corps humain ou animal positionné face à la source de rayonnement, le procédé comportant :
- une étape d'estimation, pour chaque source de rayonnement, de la distance séparant la source de rayonnement et la partie du corps exposée à ladite source de rayonnement et
- une étape de détermination d'une intensité de l'irradiation lumineuse ou infrarouge à émettre par chaque source de rayonnement en fonction de la distance séparant ladite source de rayonnement et la partie du corps à exposer à ladite source de rayonnement.

Ainsi, lors du fonctionnement du dispositif, un rayonnement lumineux ou infrarouge est émis par chaque source de rayonnement, à une intensité déterminée lors de l'étape de détermination d'une intensité.

Dans des modes de réalisation, le procédé comporte une étape de détermination d'une position où doit être placé le corps humain ou animal lors de l'utilisation du dispositif.

Dans des modes de réalisation, la position déterminée lors de l'étape de détermination est déterminée en fonction d'une valeur mesurée lors d'une étape préalable d'estimation d'au moins une valeur représentative de la distance séparant le corps du dispositif.

Dans des modes de réalisation, le procédé comporte une étape d'indication d'une position où doit être placé le corps humain, déterminée lors de l'étape de détermination, cette étape étant réalisée par un moyen d'indication permettant d'indiquer plusieurs positions prédéterminées.

Dans des modes de réalisation, l'étape d'indication d'une position comporte une étape d'émission d'un faisceau laser configuré pour marquer une position au sol, par exemple matérialisé par une ligne où un utilisateur du dispositif doit positionner ses pieds.

Dans des modes de réalisation, l'étape d'indication d'une position comporte une étape d'affichage numérique, par exemple au moyen d'un écran configuré pour afficher une image indiquant où un utilisateur doit positionner ses pieds.

Dans des modes de réalisation, le procédé de fonctionnement d'un dispositif pour exposer un corps humain ou animal à un rayonnement lumineux ou infrarouge comporte une étape de détection de la position de l'utilisateur.

Dans des modes des réalisation, l'étape de détection d'un utilisateur comporte une détection de la position de l'utilisateur au moyen d'au moins un capteur de distance, préférentiellement au moyen d'au moins un capteur de distance utilisé lors de l'étape d'estimation de la distance séparant la source de rayonnement et la partie du corps exposée à ladite source de rayonnement.

Dans des modes des réalisation, l'étape de détection de la position de l'utilisateur comporte au moins une étape d'estimation d'une pression représentative de la présence d'un corps humain ou animal face aux sources de lumière. Préférentiellement, une pluralité de capteurs de pressions sont disposés au sol devant les sources de rayonnement et configurés pour détecter la présence du corps humain ou animal.

Dans des modes des réalisation, le procédé comporte une étape de vérification visant à déterminer si la position d'un corps humain ou animal détecté lors de l'étape de détection correspond à la position où doit être placé le corps humain ou animal lors de l'utilisation du dispositif indiquée par le moyen d'indication d'une position.

Dans des modes de réalisation, une alarme sonore, visuelle ou silencieuse est émise dans le cas où le moyen de vérification ne parvient pas à confirmer la présence d'un corps à la position prévue par le moyen d'indication.

Les buts, avantages et caractéristiques particulières du procédé objet de la présente invention étant similaires à ceux du dispositif pour exposer un corps humain ou animal à un rayonnement lumineux ou infrarouge objet de la présente invention, ils ne sont pas rappelés ici.

### BRÈVE DESCRIPTION DES FIGURES

D'autres avantages, buts et caractéristiques particulières de l'invention ressortiront de la description non limitative qui suit d'au moins un mode de réalisation particulier du dispositif pour exposer un corps humain ou animal à un rayonnement lumineux ou infrarouge ou du procédé de fonctionnement d'un tel dispositif, objets de la présente invention, en regard des dessins annexés, dans lesquels :
[Fig 1] représente, schématiquement, une vue face d'un mode de réalisation particulier d'un dispositif objet de l'invention,
[Fig 2] représente, schématiquement, une vue de profil d'un mode de réalisation particulier d'un dispositif objet de l'invention, en cours d'utilisation par un utilisateur,
[Fig 3] représente, schématiquement, une vue de profil d'un autre mode de réalisation particulier d'un dispositif objet de l'invention, en cours d'utilisation,
[Fig 4] représente, schématiquement, une vue de profil du dispositif illustré en figure 1, en cours d'utilisation et
[Fig 5] représente, sous forme de logigramme, les étapes d'un mode de réalisation particulier du procédé de fonctionnement d'un dispositif pour exposer un corps humain ou animal à un rayonnement lumineux ou infrarouge, objet de l'invention.

Les numéros de référence mentionnés sur les figures se rapportent à :
10, 20, 30 Dispositif pour exposer un corps humain ou animal à un rayonnement lumineux ou infrarouge
101, 102, 103, 104, 105, 106, 107, 108, 109, 110, 111, 112, 113, 115 Diodes électroluminescentes
121, 122, 123, 124 Capteurs de distance associé à une source lumineuse
130 Capteur de distance associé à au moyen d'émission d'un faisceau laser
135 Moyen d'émission d'un faisceau laser
340 Barre
150 Boîtier de contrôle électronique
170, 171, 172, 173, 174, 175 Capteurs de pression
260 Écran d'affichage numérique
180, 185, 190, 195, 280, 285, 290, 380, 385, 390 Panneaux de support
905, 906 Sol
910 Utilisateur du dispositif
911, 912, 913, 914 Partie du corps de l'utilisateur

Les numéros de références à 4 chiffres correspondent aux étapes du procédé de fonctionnement d'un dispositif pour exposer un corps humain ou animal à un rayonnement lumineux ou infrarouge.

### DESCRIPTION DÉTAILLÉE DE L'INVENTION

La présente description est donnée à titre non limitatif, chaque caractéristique d'un mode de réalisation pouvant être combinée à toute autre caractéristique de tout autre mode de réalisation de manière avantageuse.

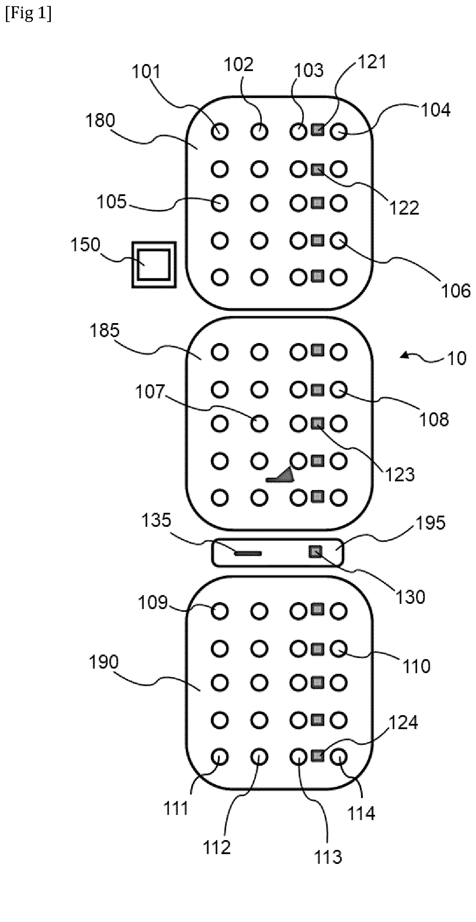

On observe en figure 1 un dispositif 10 pour exposer un corps humain ou animal à un rayonnement lumineux ou infrarouge. Le rayonnement est produit par un ensemble de diodes électroluminescentes (LED) préférentiellement configurées pour émettre un rayonnement dans une gamme de longueur d'onde comprise entre 400 nm et 950 nanomètres (nm). Par exemple, tout ou partie des LED sont configurées pour émettre un rayonnement en lumière bleue ayant une longueur d'onde comprise entre 450 nm et 490 nm, en lumière rouge ayant une longueur d'onde située entre 600 nm et 670 nm ou un rayonnement en lumière proche infrarouge ayant une longueur d'onde située entre 820 nm et 860 nm.

Le dispositif 10 comporte une pluralité de sources de rayonnement qui comportent chacune plusieurs LED et un capteur de distance.

Selon la configuration adoptée par le dispositif 10, chaque source de rayonnement comporte plusieurs LED arrangées horizontalement et un capteur de distance positionné sensiblement à la même hauteur que les LED. Ainsi, on observe les LED 101, 102, 103 et 104 qui forment, avec le capteur de distance 121, une première source de rayonnement. Cette première source de rayonnement est positionnée sur un panneau de support 180 qui est une pièce de support où sont fixées plusieurs sources de rayonnement. Le panneau 180 comporte en outre une deuxième source de rayonnement comportant le capteur de distance 122 ainsi que quatre LED, une troisième source de rayonnement comportant quatre LED dont la LED 105 et un capteur de distance, une quatrième source de rayonnement comportant quatre LED dont la LED 106 et un capteur de distance, et enfin une cinquième source de rayonnement comportant quatre LED et un capteur de distance.

Ainsi, le premier panneau 180 comporte cinq rangées de LED disposées horizontalement les unes par dessues les autres, chaque rangée étant associée à un capteur de distance et chaque rangée formant une source de rayonnement.

Selon une alternative de réalisation (non illustrée), le dispositif pour exposer un corps humain ou animal à un rayonnement lumineux ou infrarouge comporte seulement le panneau de support 180, éventuellement associé à un panneau de support 195 comportant un moyen d'indication doté d'un laser.

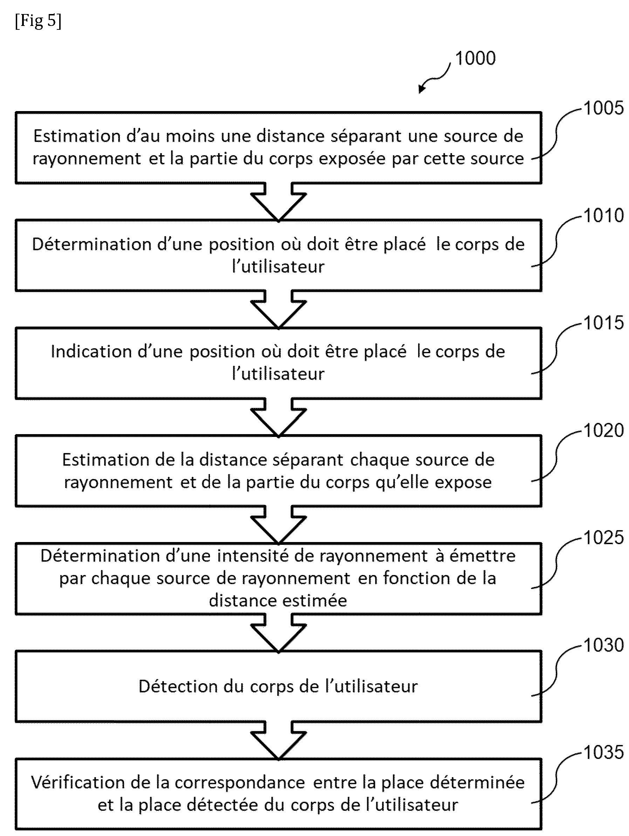

Le dispositif 10 illustré en figure 1 et en figure 5 comporte, en plus du premier panneau 180, deux panneaux supplémentaires : le deuxième panneau 185 et le troisième panneau 190, dont les caractéristiques sont identiques à celles du premier panneau 180. Le deuxième panneau 185 comporte cinq sources de rayonnement comportant, entre autres, les LED 107 et 108 et le capteur de distance 123. Le troisième panneau comporte quatre sources de rayonnement comportant, entres autres, les LED 109 et 110, et une cinquième source de rayonnement les LED 111, 112, 113 et 114 et le capteur de distance 124.

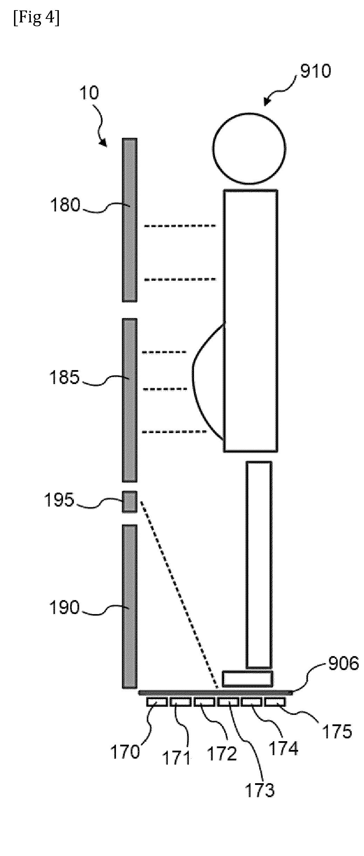

Le dispositif 10 comporte en outre un panneau 195 sur lequel sont disposés un capteur de distance 130 et un moyen d'émission d'un faisceau laser 135. Ces éléments forment ensemble un moyen d'indication d'une position où doit être placé le corps humain ou animal lors de l'utilisation du dispositif. Ces éléments seront mieux compris à la lecture de description de la figure 4.

Les panneaux 180, 185, 190 et 195 sont configurés pour être placés afin d'exposer un corps humain ou animal à la lumière émise par les LED du dispositif 10. Par exemple des moyens sont prévus pour fixer les panneaux à un mur ou à une potence. Les panneaux peuvent être reliés entre eux, articulés entre eux voire solidaires, ou être au contraire des pièces séparées.

Le dispositif 10 comporte un boîtier de contrôle électronique 150 permettant à un utilisateur d'interagir avec le dispositif 10, par exemple pour choisir un programme d'utilisation du dispositif 10, pour allumer et éteindre le dispositif 10, pour consulter des informations concernant un traitement en cours, tel que le temps écoulé, etc.

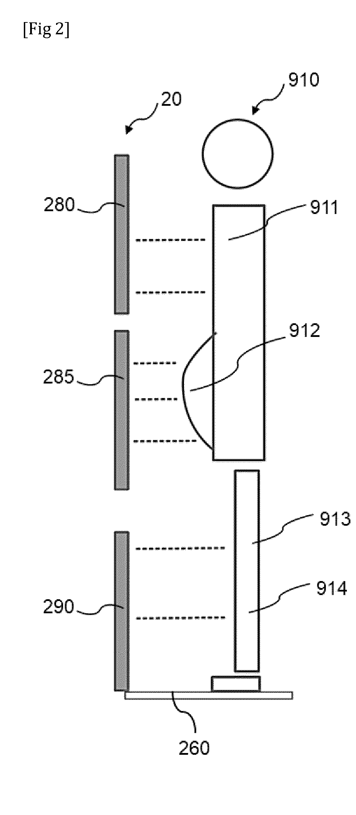

Chaque capteur de distance, 121, 122, 123, 124, est configuré pour estimer une valeur représentative de la distance séparant la source de rayonnement et la partie du corps exposée à ladite source de rayonnement. Cette mesure de distance entre l'utilisateur et la source de rayonnement est ensuite exploitée pour moduler l'intensité du rayonnement lumineux ou infrarouge émis par chaque source de rayonnement. Cette caractéristique sera mieux comprise à la lecture de la description des figures 2 et 5.

On observe en figure 2 un mode de réalisation particulier de l'invention illustrant un dispositif 20 pour exposer un corps humain ou animal à un rayonnement lumineux ou infrarouge. Le dispositif 20 comporte une pluralité de sources de rayonnement munies de LED orientées sur le corps de l'utilisateur dans des conditions normales d'utilisation. Le dispositif 20 comporte trois panneaux de support 280, 285 et 290, chacun comportant une pluralité de sources de rayonnement. Ces panneaux sont identiques aux panneaux de support 180, 185 et 190 décrits en regard de la figure 1, respectivement, ils ne sont donc pas décrits à nouveau ici.

Le dispositif 20 est représenté en cours d'utilisation. Un utilisateur adulte, 910, se tient debout face au dispositif 20. On observe que certaines sources de rayonnement illuminent le haut du corps 911 de l'utilisateur, que d'autres sources de rayonnement illuminent une région correspondant au ventre 912 de l'utilisateur et que d'autres sources de rayonnement illuminent le haut et le bas des jambes, 913 et 914. Le rayonnement lumineux ou infrarouge émis par certaines des sources de rayonnement est représenté par des lignes horizontales pointillées, qui émanent de LED montés sur les panneaux 280, 285 et 290.

Chaque source de rayonnement est munie d'un capteur de distance permettant d'estimer la distance séparant la source de rayonnement de la partie du corps de l'utilisateur qu'elle irradie et l'intensité lumineuse émise par chaque source de rayonnement est commandée en fonction de la distance estimée. Par exemple si un traitement de photobiomodulation nécessite une illumination uniforme de plusieurs parties du corps de l'utilisateur, l'intensité lumineuse émise par les sources de rayonnement qui se trouvent proches des parties du corps de l'utilisateur qu'elles exposent sera moindre que l'intensité lumineuse des sources de rayonnement se trouvent éloignées des parties du corps de l'utilisateur qu'elles exposent.

Le dispositif 20 comporte en outre un moyen d'indication d'une position où doit se placer l'utilisateur lors de l'utilisation du dispositif 20. Ce moyen d'indication comporte un écran numérique 260 se trouvant au pieds de l'utilisateur et configuré pour afficher une image informant l'utilisateur de l'endroit où il doit positionner ses pieds pour permettre une utilisation optimisée du dispositif 20. Par exemple, l'écran 260 affiche une image montrant deux empreintes de pieds, sur lesquelles l'utilisateur est invité à positionner ses pieds lors de l'utilisation du dispositif. La position affichée sur l'écran est mobile, c'est-à-dire qu'elle permet d'indiquer plusieurs positions prédéterminées, par exemple des positions préenregistrées en fonction d'un programme de traitement sélectionné.

Avantageusement, la position indiquée par le moyen d'indication d'une position est déterminée en fonction d'au moins une valeur représentative de la distance séparant le corps ou une partie du corps, du dispositif 20. Par exemple, une distance est estimée lors d'une étape d'initialisation comportant une estimation de distance par plusieurs capteurs de distance du dispositif 20. Ces distances estimées sont représentatives de la corpulence de l'utilisateur. Ces distances estimées sont ensuite utilisées pour calculer une position optimale que doit occuper l'utilisateur lors de la suite du traitement. Ce calcul peut être réalisé au moyen d'un microprocesseur inclus au dispositif 20 ou distant.

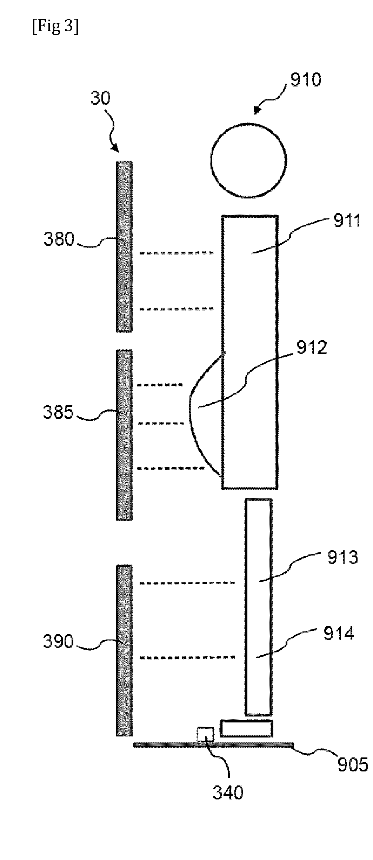

On observe en figure 3 un autre mode de réalisation particulier de l'invention illustrant un dispositif 30 pour exposer un corps humain ou animal à un rayonnement lumineux ou infrarouge. Le dispositif 30 comporte une pluralité de sources de rayonnement munies de LED orientées sur le corps de l'utilisateur dans des conditions normales d'utilisation. Le dispositif 30 comporte trois panneaux de support 380, 385 et 390, chacun comportant une pluralité de sources de rayonnement, ces panneaux sont identiques aux panneaux de support 180, 185 et 190 décrits en regard de la figure 1, respectivement ; ils ne sont donc pas décrits à nouveau ici.

Le dispositif 30 comporte un moyen d'indication d'une position où doit se placer l'utilisateur lors de l'utilisation du dispositif 30. Ce moyen comporte une barre 340 au sol 905, destinée à indiquer à l'utilisateur 910 où positionner ses pieds. Avantageusement, la barre 340 est amovible et comporte plusieurs points de fixation sur le sol 905. Alternativement, la barre 340 est fixé à une glissière. Dans ce cas, il peut être prévu un moyen de motorisation pour déplacer la barre 340 sans intervention de l'utilisateur.

On observe en figure 4 une vue de côté du dispositif 10 déjà partiellement décrit en regard de la figure 1. Le dispositif 10 comporte un moyen d'indication d'une position où doit se placer l'utilisateur lors de l'utilisation du dispositif 10. Ce moyen comporte un moyen 135 d'émission d'un faisceau laser configuré pour marquer une position, par exemple matérialisé par une ligne projetée au sol où un utilisateur du dispositif 10 est invité à positionner ses pieds. Ce faisceau est illustré en figure 4 par une barre pointillée oblique. Le faisceau laser émet une lumière visible projetée au sol, il est particulièrement avantageux car il permet de projeter une ligne bien visible et nette. De plus l'émetteur de faisceau laser est un élément compact.

Avantageusement, la position indiquée au sol par le faisceau laser est déterminée en fonction d'au moins une valeur représentative de la distance séparant le corps ou une partie du corps de l'utilisateur. Cette distance peut être estimée par le capteur de distance 130, visible en figure 1.

Le dispositif 10 comporte en outre un moyen de détection de la position de l'utilisateur. Ce moyen de détection comporte une pluralité de capteurs de pression 170, 171, 172, 173, 174, disposés au sol et recouvert d'une couverture souple 905 tenant lieu de sol. Les capteurs de pression sont configurés pour détecter la présence de l'utilisateur en détectant son poids.

Selon une alternative de réalisation (non représenté) le dispositif comporte un unique capteur de pression. Dans ce cas seule la présence ou l'absence de l'utilisateur peut être vérifiée. Si un poids est détecté, cela confirme la présence d'un utilisateur.

Selon le mode de réalisation illustré en figure 4, la pluralité de capteur de pression, 170, 171, 172, 173 et 174 permet de détecter la présence de l'utilisateur mais également d'estimer sa position, plus ou moins proche des sources de rayonnement portées par les panneaux 180, 185 et 190.

Selon un mode de réalisation alternatif, le moyen de détection de la position de l'utilisateur détecte la position de l'utilisateur au moyen d'au moins de l'un des capteurs de distance, 121, 122, 123, 124, 130, du dispositif 10.

Avantageusement, le dispositif 10 comporte un moyen de vérification configuré pour déterminer si un utilisateur détecté par un moyen de détection est en effet présent et éventuellement si sa position correspond à la position où doit être placé l'utilisateur. Cette position où doit être placé l'utilisateur étant montrée à l'utilisateur par un moyen d'indication tel que décrit précédemment. Dans le cas où le moyen de vérification ne parvient pas à confirmer la présence d'un corps à la position prévue une alarme sonore, visuelle ou silencieuse peut être émise.

Avantageusement, l'intensité de l'irradiation lumineuse ou infrarouge émise par au moins une source de rayonnement est déterminée en fonction de la vérification opérée par le moyen de vérification. Par exemple, dans le cas où le moyen de vérification ne parvient pas à confirmer la présence d'un corps à la position prévue par le moyen d'indication, l'intensité de l'irradiation lumineuse ou infrarouge émise par au moins une source de rayonnement est réduite à une très basse intensité ou est éteinte.

On observe en figure 5, un logigramme du procédé 1000 de fonctionnement d'un dispositif pour exposer un corps humain ou animal à un rayonnement lumineux ou infrarouge par exemple un dispositif 10, 20 ou 30 décrit précédemment.

Dans des modes de réalisation, le procédé 1000 comporte une étape 1005 d'estimation d'au moins une valeur représentative de la distance séparant le corps du dispositif.

Dans des modes de réalisation, le procédé 1000 comporte une étape de détermination 1010 d'une position où doit être placé le corps humain ou animal lors de l'utilisation du dispositif.

Dans des modes de réalisation, la position où doit être positionné le corps humain ou animal est calculée en fonction de la mesure de distance réalisée lors de l'étape 1005. La position devant être indiquée par le moyen d'indication d'une position est déterminée en fonction d'au moins une valeur représentative de la distance séparant le corps, ou une partie du corps, du dispositif. D'autres paramètres incluant le type de traitement sélectionné, le sexe de l'utilisateur, la carnation, ou tout autre information physiologique renseignée par l'utilisateur ou mesurée aux moyens de capteurs additionnels peuvent également être pris en compte pour le calcul de la distance. Dans des modes de réalisation, la position où doit être positionné le corps humain ou animal est calculée au début d'un traitement, mise en mémoire et conservée durant tous le traitement. Dans d'autres modes de réalisation, la position où doit être positionné le corps humain ou animal est calculée plusieurs fois lors du fonctionnement du dispositif, par exemple à intervalle de temps réguliers. Dans ce dernier cas, les étapes 1010 et 1015 sont répétés plusieurs fois lors du procédé.

Lors d'une étape d'indication 1015, un moyen d'indication est mis en œuvre pour informer l'utilisateur de l'endroit où il doit se placer par rapport au dispositif. Comme décrit précédemment, le moyen d'indication est par exemple un faisceau laser formant une ligne ou une marque sur le sol. Selon un autre exemple, le moyen d'indication est un écran d'affichage.

Le procédé 1000 comporte une étape 1020 d'estimation, pour chaque source de rayonnement, de la distance séparant la source de rayonnement et la partie du corps exposée à ladite source de rayonnement. Le procédé 1000 comprend par ailleurs une étape 1025 de détermination d'une intensité de l'irradiation lumineuse ou infrarouge à émettre par chaque source de rayonnement en fonction de la distance séparant ladite source de rayonnement et la partie du corps exposée à ladite source de rayonnement. Ainsi, lors du fonctionnement du dispositif, un rayonnement lumineux ou infrarouge est émis par chaque source de rayonnement, à une intensité déterminée lors de l'étape de détermination d'une intensité.

Dans des modes de réalisation, le procédé 1000 comporte une étape 1030 de détection de la position de l'utilisateur. Comme décrit précédemment, l'étape 1030 peut par exemple être réalisée au moyen d'un capteur de distance ou au moyen d'au moins un capteur de pression.

Dans des modes de réalisation, le procédé 1000 comporte une étape de vérification 1035 visant à déterminer si la position de l'utilisateur détecté lors de l'étape 1030 de détection correspond à la position indiquée lors de l'étape 1015 d'indication d'une position. Comme décrit précédemment une alarme peut être émise dans le cas où la présence de l'utilisateur n'est pas vérifiée, ou l'utilisateur peut être invité à ajuster sa position par un message sonore ou affiché sur un écran. De plus, si la présence d'un utilisateur n'est pas détectée, une commande peut être envoyée aux sources de rayonnement pour l'éteindre. De même, une commande d'extinction des LED ou d'atténuation de l'intensité lumineuse qu'elle émettent peut être émise lorsque l'utilisateur est détecté trop proche des sources de rayonnement.

## Revendications

1. Dispositif (10, 20, 30) pour exposer un corps humain ou animal à un rayonnement lumineux ou infrarouge, **caractérisé en ce qu'**il comporte une pluralité de sources de rayonnement, chacune comportant au moins une diode électroluminescente (101, 102, 103, 104, 105, 106, 107, 108, 109, 110, 111, 112, 113, 115) disposée pour exposer à un rayonnement une partie du corps (911, 912, 913, 914) humain ou animal positionnée face à la source de rayonnement,
dans lequel chaque source de rayonnement, comporte un capteur de distance (121, 122, 123, 124) configuré pour estimer une valeur représentative de la distance séparant la source de rayonnement et la partie du corps exposée à ladite source de rayonnement,
et dans lequel l'intensité de l'irradiation lumineuse ou infrarouge émise par chaque source de rayonnement est déterminée en fonction de la distance séparant ladite source de rayonnement et la partie du corps exposée à ladite source de rayonnement.

2. Dispositif (10, 20, 30) selon la revendication 1, qui comporte un moyen d'indication (135, 260, 340) d'une position où doit être placé le corps humain ou animal lors de l'utilisation du dispositif et dans lequel la position indiquée par le moyen d'indication d'une position est mobile, permettant d'indiquer plusieurs positions prédéterminées.

3. Dispositif (10, 20, 30) selon la revendication 2, dans lequel la position indiquée par le moyen d'indication d'une position est déterminée en fonction d'au moins une valeur représentative de la distance séparant le corps ou une partie du corps, du dispositif.

4. Dispositif (10) selon l'une des revendications 2 ou 3, dans lequel le moyen d'indication d'une position comporte un moyen (135) d'émission d'un faisceau laser configuré pour marquer une position, par exemple matérialisé par une ligne projetée au sol où un utilisateur du dispositif doit positionner ses pieds.

5. Dispositif (20) selon l'une des revendications 3 à 4, dans lequel le moyen d'indication d'une position comporte un moyen d'affichage numérique (260), préférentiellement un écran ou un projecteur configuré pour afficher une image indiquant où un utilisateur du dispositif doit positionner ses pieds.

6. Dispositif (10, 20, 30) selon l'une des revendications 1 à 5, qui comporte un moyen de détection de la position de l'utilisateur.

7. Dispositif (10, 20, 30) selon la revendication 6, dans lequel le moyen de détection de la position de l'utilisateur détecte la position de l'utilisateur au moyen d'au moins un capteur de distance (121, 122, 123, 124, 130), préférentiellement au moyen de l'un au moins des capteurs de distance des sources de lumière.

8. Dispositif (10, 20, 30) selon l'une des revendications 6 ou 7, dans lequel le moyen de détection de la position de l'utilisateur comporte au moins un capteur de pression (170, 171, 172, 173, 174, 175), préférentiellement une pluralité de capteurs de pressions, disposé au sol devant les sources de rayonnement et configuré pour détecter la présence du corps humain ou animal.

9. Dispositif (10, 20, 30) selon l'une des revendications 6 à 8, lorsqu'elles dépendent de l'une des revendications 2 à 5, qui comporte un moyen de vérification configuré pour déterminer si la position d'un corps humain ou animal détectée par le moyen de détection correspond à la position où doit être placé le corps humain ou animal lors de l'utilisation du dispositif indiquée par le moyen d'indication d'une position.

10. Procédé (1000) de fonctionnement d'un dispositif pour exposer un corps humain ou animal à un rayonnement lumineux ou infrarouge comportant une pluralité de sources de rayonnement, chacune comportant au moins une diode électroluminescente disposée pour exposer à un rayonnement une partie du corps humain ou animal positionné face à la source de rayonnement,
**caractérisé en ce que** qu'il comporte :
- une étape (1020) d'estimation, pour chaque source de rayonnement, de la distance séparant la source de rayonnement et la partie du corps exposée à ladite source de rayonnement et
- une étape (1025) de détermination d'une intensité de l'irradiation lumineuse ou infrarouge à émettre par chaque source de rayonnement en fonction de la distance séparant ladite source de rayonnement et la partie du corps exposée à ladite source de rayonnement.

11. Procédé (1000) selon la revendication 10, qui comporte en outre :
- une étape (1005) d'estimation d'au moins une valeur représentative de la distance séparant le corps et au moins une source de rayonnement et
- une étape (1010) de détermination d'une position où doit être placé le corps humain ou animal lors de l'utilisation du dispositif
- une étape (1015) d'indication durant laquelle un moyen d'indication est mis en œuvre pour informer l'utilisateur de l'endroit où il doit se placer par rapport au dispositif
et dans lequel la position déterminée lors de l'étape (1010) de détermination d'une position est calculée en fonction d'au moins une valeur représentative de la distance séparant le corps et au moins une source de rayonnement estimée lors de l'étape (1005) d'estimation.

12. Procédé (1000) selon la revendication 10 ou 11, dans lequel la position indiquée par le moyen d'indication d'une position est mobile, permettant d'indiquer plusieurs positions prédéterminées.

13. Procédé (1000) selon l'une des revendication 10 à 12 qui comporte en outre :
- une étape (1030) de détection de la position de l'utilisateur et
- une étape (1035) de vérification 1035 visant à déterminer si la position de l'utilisateur détecté lors de l'étape (1030) de détection correspond à la position indiquée lors de l'étape (1015) d'indication d'une position.

14. Procédé (1000) selon la revendication 13 dans lequel une alarme est émise dans le cas où la présence de l'utilisateur n'est pas détectée, ou dans le cas où un utilisateur est détecté dans une position distincte de celle déterminée lors de l'étape (1010) de détermination d'une position où doit être placé le corps humain ou animal lors de l'utilisation du dispositif.
